# EUROPEAN PATENT SPECIFICATION

(11) **EP 2 139 866 B1**
(45) Date of publication and mention of the grant of the patent: **12.01.2011**
(21) Application number: 08749704.6
(22) Date of filing: 24.04.2008
(51) Int. Cl.: C07D 235/26, A61K 31/4184, A61P 9/00

(54) **CRYSTALLINE 1-(CYCLOHEXYLOXYCARBONYLOXY) ETHYL 1-((2'-CYANOBIPHENYL-4-YL)METHYL)-2-ETHOXY-1H-BENZO[D]IMIDAZOLE-7-CARBOXYLATE AND A PROCESS FOR ITS PREPARATION**
KRISTALLINES 1-(CYCLOHEXYLOXYCARBONYLOXY)-ETHYL 1-((2'-CYANOBIPHENYL-4-YL)-METHYL)-2-ETHOXY-1H-BENZO-[D]-IMIDAZOL-7-CARBOXYLAT UND VERFAHREN ZU SEINER HERSTELLUNG
1-(CYCLOHÉXYLOXYCARBONYLOXY) ÉTHYL 1-((2'-CYANOBIPHÉNYL-4-YL)MÉTHYL)-2-ÉTHOXY-1H-BENZO[D]IMIDAZOLE-7-CARBOXYLATE CRISTALLIN, ET SON PROCÉDÉ DE PRÉPARATION

(30) Priority: 24.04.2007 SI 200700100
(43) Date of publication of application: 06.01.2010
(73) Proprietor: KRKA, D.D., Novo Mesto, 8501 Novo mesto (SI)
(72) Inventor: ZUPANCIC, Silvo, 8000 Novo Mesto (SI); SMRKOLJ, Matej, 1420 Trbovlje (SI); OSOLNIK, Renata, 8361 Straza (SI); VRBINC, Miha, 8000 Novo Mesto (SI)
(74) Representative: HOFFMANN EITLE
(86) International application number: PCT/EP2008/054997
(87) International publication number: WO 2008/129077

(56) References cited:
- EP-A- 0 459 136
- WO-A-2006/023889
- WO-A-2007/039117
- CN-A- 1 361 101
- CN-A- 1 425 654
- ANDERSON ET AL: "TOOLS FOR PURIFYING THE PRODUCT: COLUMN CHROMATOGRAPHY, CRYSTALLIZATION AND RESLURRYING" PRACTICAL PROCESS RESEARCH AND DEVELOPMENT, ACADEMIC PRESS, SAN DIEGO, US, 1 January 2000 (2000-01-01), pages 223-247, XP002382432
- NDA of Atacand(RTM) 32 mg tablets
- German "Fachinformation" of Atcand(RTM) Protect 32 mg tablets

## Description

### Field of the invention

The present invention relates to 1-(cyclohexyloxycarbonyloxy)ethyl 1-((2'-cyanobiphenyl-4-yl)methyl)-2-ethoxy-1*H*-benzo[*d*]imidazole-7-carboxylate in crystalline form and a process for its preparation, which is useful intermediate in the preparation of candesartan cilexetil. The present invention also relates to the preparation of candesartan cilexetil and pharmaceutical composition comprising candesartan cilexetil.

### Background of the invention

The preparation of 1-(cyclohexyloxycarbonyloxy)ethyl 1-((2'-cyanobiphenyl-4-yl)methyl)-2-ethoxy-1*H-*benzo[*d*]imidazole-7-carboxylate with the structural formula (I): 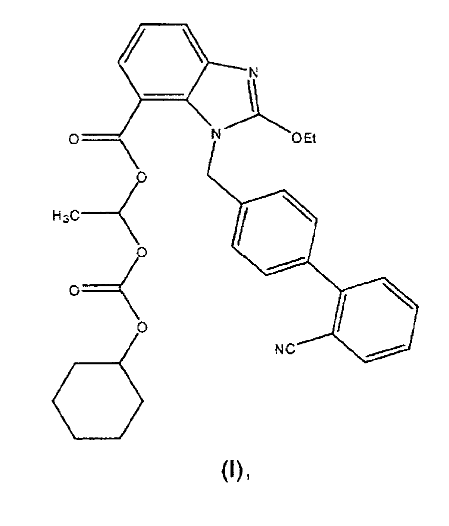 has already been described by the Chinese patent application CN 1361101 A, Examples 8-10. 1-[(2'-Cyanobiphenyl-4-yl)methyl]-2-ethoxy-1*H*-benzimidazole-7-carboxylic acid is reacted with cyclohexyl1-iodoethyl carbonate in DMF under basic conditions. A ropy resinoid compound was obtained from EtOAc, due to the presence of impurities and solvents in the product.

Further on, the preparation of 1-(cyclohexyloxycarbonyloxy)ethyl 1-((2'-cyanobiphenyl-4-yl)methyl)-2-ethoxy-1*H*-benzo[*d*]imidazole-7-carboxylate has also been disclosed in WO 2007/039117, Example 9. Potassium (2-cyanophenyl)-tetrafluoroboronate was reacted with 1-[[(cyclohexyloxy)carbonyl]oxy]ethyl 1-(4-bromobenzyl)-2-ethoxy-1*H*-benzimidazole-7-carboxylate in the presence of Pd acetate and tri-*o*-tolylphosphine. Oily 1-(cyclohexyloxycarbonyloxy)ethyl 1-((2'-cyanobiphenyl-4-yl)methyl)-2-ethoxy-1*H-*benzo[*d*]imidazole-7-carboxylate was isolated, and reacted further to yield candesartan cilexetil, an antihypertensive from the group of sartans. Due to the persistent presence of impurities the candesartan cilexetil obtained was also in oily form.

The drawback of the prior art processes is that 1-(cyclohexyloxycarbonyloxy)ethyl 1-((2'-cyanobiphenyl-4-yl)methyl)-2-ethoxy-1*H*-benzo[*d*]imidazole-7-carboxylate is isolated in oily-resinoid form which is difficult to purify and difficult to handle when being used in the preparation of candesartan cilexetil.

The technical problem the present invention was trying to solve was the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate which enables a straightforward and efficient transformation into candesartan cilexetil of formula (II): 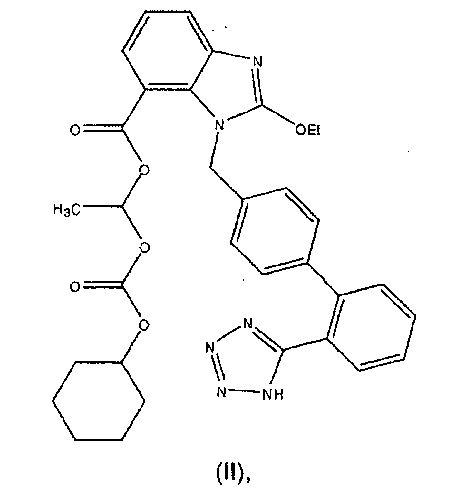 without the presence of persisting impurities originating from oily starting compound.

The object is achieved by the preparation of crystalline form of 1-(cyclohexyloxycarbonyloxy)ethyl 1-((2'-cyanobiphenyl-4-yl)methyl-2-ethoxy-1*H-*benzo[*d*]imidazole-7-carboxylate according to the invention, and the process for its preparation and its transformation into candesartan cilexetil.

### Brief description of drawings

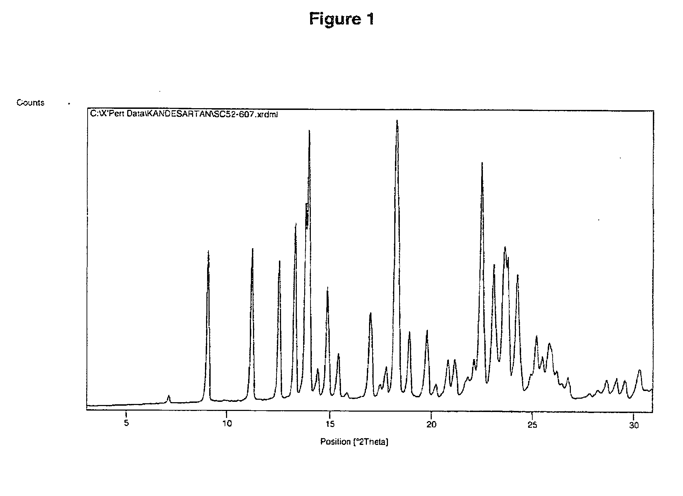

Figure 1 discloses an X-ray powder diffraction spectrum of crystalline 1-(cyclohexyloxycarbonyloxy)ethyl 1-((2'-cyanobiphenyl-4-yl)methyl)-2-ethoxy-1*H-*benzo[*d*]imidazole-7-carboxylate according to the invention.

### Summary of the invention

According to the first aspect of the present invention there is provided a crystalline form of 1-(cyclohexyloxycarbonyloxy)ethyl 1-((2'-cyanobiphenyl-4-yl)methyl-2-ethoxy-1*H-*benzo[*d*]imidazole-7-carboxylate characterized by an X-ray powder diffraction pattern containing the following 2-theta peaks measured using CuKα radiation: 9.1, 11.2, 13.3, 14.0, 18.4, 22.5, 23.6 ± 0.2 degrees two-theta.

According to the second aspect of the invention there is provided a process for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising: a) dissolving of 1-(cyclohexyloxycarbonyloxy)ethyl 1-((2'-cyanobiphenyl-4-yl)methyl)-2-ethoxy-1*H*-benzo[*d*]imidazole-7-carboxylate in form of an oily residue in an unpolar organic solvent and b) optionally heating the mixture followed by cooling the solution, c) crystallization and isolating the final product.

According to the third aspect of the invention there is provided a process for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising: a) reacting 1-[[(cyclohexyloxy)carbonyl]oxy]ethyl 1-(4-bromobenzyl)-2-ethoxy-1*H*-benzimidazole-7-carboxylate with 2-cyanophenylboronic acid or its derivative in an organic solvent by an addition of a catalyst to obtain 1-(cyclohexyloxycarbonyloxy)ethyl 1-((2'-cyanobiphenyl-4-yl)methyl)-2-ethoxy-1*H-*benzo[*d*]imidazole-7-carboxylate in the form of a solution or an oily residue, b) dissolving of the obtained 1-(cyclohexyloxycarbonyloxy)ethyl 1-((2'-cyanobiphenyl-4-yl)methyl)-2-ethoxy-1*H*-benzo[*d*]imidazole-7-carboxylate in an unpolar organic solvent, c) optionally heating the mixture followed by cooling the solution and d) crystallization and isolating the final product.

According to the fourth aspect of the invention there is provided a process for the preparation of crystalline form of 1-(cyclohexyloxycarbonyloxy)ethyl 1-((2'-cyanobiphenyl-4-yl)methyl)-2-ethoxy-1H-benzo[d]imidazole-7-carboxylate comprising: a) reacting an ester of 2-Ethoxy-1-[(2'-cyanobiphenyl-4-yl)-methyl]-1H-benzimidazole-7-carboxylic acid with a base to form a corresponding salt of 2-Ethoxy-1-[(2'-cyanobiphenyl-4-yl)-methyl]-1H-benzimidazole-7-carboxylic acid, b) reacting a salt of 2-Ethoxy-1-[(2'-cyanobiphenyl-4-yl)-methyl]-1H-benzimidazole-7-carboxylic acid with an acid to form 2-Ethoxy-1-[(2'-cyanobiphenyl-4-yl)-methyl]-1H-benzimidazole-7-carboxylic acid and c) Reacting 2-Ethoxy-1-[(2'-cyanobiphenyl-4-yl)-methyl]-1H-benzimidazole-7-carboxylic acid with 1-haloethyl cyclohexyl carbonate to form final product.

According to the fifth aspect of the invention there is provided the use of a crystalline form of 1-(cyclohexyloxycarbonyloxy)ethyl 1-((2'-cyanobiphenyl-4-yl)methyl)-2-ethoxy-1*H-*benzo[*d*]imidazole-7-carboxylate for the preparation of candesartan cilexetil or a solid pharmaceutical composition containing candesartan cilexetil.

According to the sixth aspect of the invention there is provided a process for the preparation of candesartan cilexetil comprising a) preparartion of a crystalline form of 1-(cyclohexyloxycarbonyloxy)ethyl 1-((2'-cyanobiphenyl-4-yl)methyl-2-ethoxy-1*H-*benzo[*d*]imidazole-7-carboxylate, b) cycloaddition of an azide on said crystalline 1-(cyclohexyloxycarbonyloxy)ethyl 1-((2'-cyanobiphenyl-4-yl)methyl-2-ethoxy-1*H-*benzo[*d*]imidazole-7-carboxylate to obtain crude candesartan cilexetil, c) purification of crude candesartan cilexetil and d) crystallization of candesartan cilxetil.

In another aspect of the present invention candesartan cilexetil prepared by the process according to the present invention is formulated into a stable solid pharmaceutical composition wherein the granules of candesartan cilexetil and at least one pharmaceutically acceptable ingredient are preferably prepared in a fluid granulator and wherein candesartan cilexetii is dispersed in a solvent.

In yet another aspect of the present invention, there is provided a process for the preparation of stable solid pharmaceutical composition containing candesartan cilexetil, wherein the phamarceutical composition is for use in a method of treating hypertension, cardiac diseases, cerebral apoplexy or renal diseases in a patient in need thereof, and wherein the granules of the active substance and at least one pharmaceutically acceptable ingredient are preferably prepared in a fluid-bed granulator and wherein candesartan cilexetil is dispersed in a solvent.

### Description of the invention

According to the first aspect of the present invention there is provided a crystalline form of 1-(cyclohexyloxycarbonyloxy)ethyl 1-((2'-cyanobiphenyl-4-yl)methyl-2-ethoxy-1*H*-benzo[*d*]imidazole-7-carboxylate characterized by an X-ray powder diffraction pattern, ± 0.2 degrees two-theta, expressed in Table I and Figure 1, measured using CuK_{α} radiation:

**Table I**

| **Position of diffraction lines and relative intensities** | | | |
|---|---|---|---|
| **No.** | **Pos.** **[°2Th.]** | **d-spacing** **[A]** | **Rel. Int.** **[%]** |
| | | | |
| 1 | 9.1 | 9.74 | 55 |
| 2 | 11.2 | 7.92 | 54 |
| 3 | 13.3 | 6.67 | 63 |
| 4 | 14.0 | 6.34 | 96 |
| 5 | 18.4 | 4.83 | 100 |
| 6 | 22.5 | 3.95 | 85 |
| 7 | 23.6 | 3.77 | 57 |

Preferably, the crystalline form of 1-(cyclohexyloxycarbonyloxy)ethyl 1-((2'-cyanoblphonyl-4-yl)methyl-2-ethoxy-1*H*-benzo[*d*]imidazole-7-carboxylate prepared according to the present invention is characterized by the X-ray powder diffraction pattern expressed in Table II, measured using CuK_{α} radiation:

**Table II**

| **Position of diffraction lines and relative intensities** | | | |
|---|---|---|---|
| **No.** | **Pos.** **[°2Th.]** | **d-spacing** **[A]** | **Rel. Int.** **[%]** |
| | | | |
| 1 | 9.1 | 9.74 | 55 |
| 2 | 11.2 | 7.92 | 54 |
| 3 | 12.5 | 7.08 | 51 |
| 4 | 13.3 | 6.67 | 63 |
| 5 | 13.8 | 6.42 | 65 |
| 6 | 14.0 | 6.34 | 96 |
| 7 | 14.9 | 5.95 | 42 |
| 8 | 15.4 | 5.75 | 19 |
| 9 | 17.0 | 5.21 | 34 |
| 10 | 18.4 | 4.83 | 100 |
| 11 | 19.0 | 4.68 | 27 |
| 12 | 19.8 | 4.48 | 27 |
| 13 | 22.5 | 3.95 | 85 |
| 14 | 23.1 | 3.85 | 51 |
| 15 | 23.6 | 3.77 | 57 |
| 16 | 23.8 | 3.74 | 51 |
| 17 | 24.2 | 3.67 | 48 |

The powder diffraction pattern of the new crystalline form of 1-(cyclohexyloxycarbonyloxy)ethyl 1-((2'-cyanobiphenyl-4-yl)methyl-2-ethoxy-1*H-*benzo[*d*]imidazole-7-carboxylate is determined under the following conditions:

| | |
|---|---|
| Equipment: | Phillips PW3040/60 X'Pert PRO diffractometer |
| Radiation: | CuK_{α} radiation (λ: 1.541874 Á) |
| Exciting voltage: | 45 kV |
| Anode current: | 40 mA |
| Sample: | Back filled sample holder, measured at room temperature. |

IR spectra were taken on a Paragon 100 Perkin-Elmer FT-IR spectrometer.

According to the second aspect of the invention there is provided a process for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising:
a) dissolving of 1-(cyclohexyloxycarbonyloxy)ethyl 1-((2'-cyanobiphenyl-4-yl)methyl)-2-ethoxy-1*H*-benzo[*d*]imidazole-7-carboxylate prepared according to known processes (as for example disclosed in EP 0 459 136) in form of an oily residue in an unpolar organic solvent,
b) optionally heating the mixture to a temperature between 20 to 120°C, preferably between 20 and 40°C, for 0 to 24 hours, preferably for 0 to 5 hours, followed by cooling the solution to a temperature between -15°C and room temperature, preferably between 0 to 15°C,
c) crystallization and isolating the crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate.

As the unpolar organic solvent solvents such as for example ethers (such as for example diethyl ether, diisopropyl ether, *tert*-butyl methyl ether and like), cyclohexane, alkanes, halogenated alkanes, or any mixtures thereof, can be used. It is preferred to use ethers such as for example tert-butyl methyl ether.

The crystals are isolated by filtration or centrifuging, dried and optionally milled. Drying may be achieved for example via application of heat, preferably carried out under ambient or reduced pressure or via contacting 1-(cyclohexyloxycarbonyloxy)ethyl 1-((2'-cyanobiphenyl-4-yl)methyl)-2-ethoxy-1*H-*benzo[*d*]imidazole-7-carboxylate with humid air in a fluidized bed drier, wherein the atmosphere in the fluidized bed drier is at least 15% humidity. Preferably, vacuum drying in a commercially available vacuum dryer is applied.

According to the third aspect of the invention there is provided a process for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising:
a) reacting 1-[[(cyclohexyloxy)carbonyl]oxy]ethyl 1-(4-bromobenzyl)-2-ethoxy-1*H-*benzimidazole-7-carboxylate prepared according to known processes (as for example disclosed in CN1970554, CN1510031, WO2006134078, WO2006063578) with 2-cyanophenylboronic acid or its derivative as for example potassium 2-cyanotrifluoroboronate in an organic solvent by an addition of a catalyst to obtain 1-(cyclohexyloxycarbonyloxy)ethyl 1-((2'-cyanobiphenyl-4-yl)methyl)-2-ethoxy-1*H*-benzo[*d*]imidazole-7-carboxylate in the form of a solution or an oily residue,
b) dissolving of the obtained 1-(cyclohexyloxycarbonyloxy)ethyl 1-((2'-cyanobiphenyl-4-yl)methyl)-2-ethoxy-1*H*-benzo[*d*]imidazole-7-carboxylate in an unpolar organic solvent,
c) optionally heating the mixture to a temperature between 20 to 120°C, preferably between 20 and 40°C, for 0 to 24 hours, preferably for 0 to 5 hours, followed by cooling the solution to a temperature between -15°C and room temperature, preferably between 0 to 15°C,
d) crystallization and isolating the crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate.

An organic solvent used in step a) could be selected from the group consisting of alcohols such as for example ethanol, or ethers such as for example 1,4-dioxane or any mixtures thereof.

A catalyst used in step a) could be selected from palladium catalysts such as for example Palladium complexes.

As the unpolar organic solvent solvents such as for example ethers (such as for example diethyl ether, diisopropyl ether, *tert*-butyl methyl ether and like), cyclohexane, alkanes, halogenated alkanes, or any mixtures thereof, can be used. It is preferred to use ethers such as for example tert-butyl methyl ether.

The crystals are isolated by filtration or centrifuging, dried and optionally milled. Drying may be achieved for example via application of heat, preferably carried out under ambient or reduced pressure or via contacting 1-(cyclohexyloxycarbonyloxy)ethyl 1-((2'-cyanobiphenyl-4-yl)methyl)-2-ethoxy-1*H-*benzo[*d*]imidazole-7-carboxylate with humid air in a fluidized bed drier, wherein the atmosphere in the fluidized bed drier is at least 15% humidity. Preferably, vacuum drying in a commercially available vacuum dryer is applied.

According to the fourth aspect of the invention there is provided a process for the preparation of crystalline form of 1-(cyclohexyloxycarbonyloxy)ethyl 1-((2'-cyanobiphenyl-4-yl)methyl)-2-ethoxy-1H-benzo[d]imidazole-7-carboxylate comprising:
a) reacting an ester of 2-Ethoxy-1-[(2'-cyanobiphenyl-4-yl)-methyl]-1H-benzimidazole-7-carboxylic acid prepared according to known processes (as for example disclosed in EP 0 459 136 patent) with a base to form a corresponding salt of 2-Ethoxy-1-[(2'-cyanobiphenyl-4-yl)-methyl]-1H-benzimidazole-7-carboxylic acid,
b) reacting a salt of 2-Ethoxy-1-[(2'-cyanobiphenyl-4-yl)-methyl]-1H-benzimidazole-7-carboxylic acid with an acid to form 2-Ethoxy-1-[(2'-cyanobiphenyl-4-yl)-methyl]-1H-benzimidazole-7-carboxylic acid,
c) reacting 2-Ethoxy-1-[(2'-cyanobiphenyl-4-yl)-methyl]-1H-benzimidazole-7-carboxylic acid with 1-haloethyl cyclohexyl carbonate to form 1-(cyclohexyloxycarbonyloxy)ethyl 1-((2'-cyanobiphenyl-4-yl)methyl)-2-ethoxy-1H-benzo[d]imidazole-7- carboxylate,
d) dissolving of the obtained 1-(cyclohexyloxycarbonyloxy)ethyl 1-((2'-cyanobiphenyl-4-yl)methyl)-2-ethoxy-1*H*-benzo[*d*]imidazole-7-carboxylate in an unpolar organic solvent,
e) optionally heating the mixture to a temperature between 20 to 120°C for 0 to 24 hours, followed by cooling the solution to a temperature between -15°C and room temperature,
f) crystallization and isolating the crystalline form of 1-(cyclohexyloxycarbonyloxy)ethyl 1-((2'-cyanobiphenyl-4-yl)methyl)-2-ethoxy-1*H-*benzo[*d*]imidazole-7-carboxylate.

A base used in the step a) could be selected from the group consisting of, but not limited to, NaOH, KOH, LiOH, Mg(OH)₂ or Ca(OH)₂ , preferably aqueous solutions of KOH and NaOH might be used. The starting ester compound could be alkyl, preferably methyl or ethyl ester.

An acid used in the step b) could be selected from the group consisting of, but not limited to, HCl, H₂SO₄, H₃PO₄ and organic acids, preferably HCl might be used.

Haloethyl cyclohexyl carbonate used in step c) could be selected from the group consisting of, but not limited to, chloroethyl, iodoethyl, bromoethyl, preferably chloroethyl cyclohexyl carbonate might be used.

Step c) could be performed in an organic solvent such as for example DMF, DMA, acetonitrile, DMSO, toluene, THF, 1,4-dioxane and the like and any mixtures thereof. The reaction may be catalyzed by an addition of a base such as for example K2CO3, Na2CO3, NaOH, KOH, different phosphates and the like and any organic base and any mixtures thereof. The reaction mixture could be optionally heated to a temperature between 20 to 120°C, preferably between 30 and 70°C, for 0 to 24 hours, preferably for 0 to 5 hours, followed by cooling the solution by an addition of water and organic solvent. The phases were separated, organic phase washed and evaporated to oily residue which could be crystallized from unpolar organic solvent. As the unpolar organic solvent solvents such as for example ethers (such as for example diethyl ether, diisopropyl ether, *tert*-butyl methyl ether and like), cyclohexane, alkanes, halogenated alkanes, or any mixtures thereof, can be used. It is preferred to use ethers such as for example tert-butyl methyl ether.

Average particle size of particles of crystalline form of 1-(cyclohexyloxycarbonyloxy)ethyl 1-((2'-cyanobiphenyl-4-yl)methyl-2-ethoxy-1*H-*benzo[*d*]imidazole-7-carboxylate obtained by the processes according to the present invention could be between 5 µm to 1000 µm when stirring. If unstirred, the process might also give bigger particles, such as for example with an average size of above 1000 µm which may be milled or processed in any other way which reduces particle size, prior to their application.

According to the fifth aspect of the invention there is provided the use of a crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate for the preparation of candesartan cilexetil or a solid pharmaceutical composition comprising candesartan cilexetil.

According to the sixth aspect of the invention there is provided a process for the preparation of candesartan cilexetil in any polymorphic form comprising:
a) preparartion of a crystalline form of 1-(cyclohexyloxycarbonyloxy)ethyl 1-((2'-cyanobiphenyl-4-yl)methyl-2-ethoxy-1*H*-benzo[*d*]imidazole-7-carboxylate
b) cycloaddition of an azide on said crystalline 1-(cyclohexyloxycarbonyloxy)ethyl 1-((2'-cyanobiphenyl-4-yl)methyl-2-ethoxy-1*H*-benzo[*d*]imidazole-7-carboxylate to obtain crude candesartan cilexetil,
c) purification of crude candesartan cilexetil,
d) crystallization of candesartan cilxetil.

Crystalline 1-(cyclohexyloxycarbonyloxy)ethyl 1-((2'-cyanobiphenyl-4-yl)methyl-2-ethoxy-1*H*-benzo[*d*]imidazole-7-carboxylate prepared by the processes according to the present invention can be converted to candesartan cilexetil by cycloaddition reaction using azides such as for example tributyltin azide. The reaction can be performed in an organic solvent such as for example toluene, xylene and the like at elevated temperature such as for example between 100 and 140°C for 20 to 70 hours. When the reaction is completed, the solvent is removed.

From the residue, purification of crude candesartan cilexetil could be performed by purification on column chromatography by the processes known from the prior art such as for example disclosed in patent applications WO 2008/012371 and WO 2008/012372.

Alternatively purification of crude candesartan cilexetil is carried out by tritylation and detritylation steps. Tritylation step is performed in any organic solvent such as for example dichloromethane, toluene, xylene and the like by triphenylchloromethane using a base such as for example K2CO3, Na2CO3, NaOH, KOH, different phosphates and the like and any organic base and any mixtures thereof, preferably trialkylamine might be used as a catalyst at elevated temperature such as for example between 0 and 100°C for 0 to 10 hours. After the reaction is completed, the reaction mixture is optionally worked up with silicagel and evaporated. To the residue an organic solvent such as for example ethers, for example *tert*-butylmethyl ether is added and the crystals of tritylcandesartan cilexetil are formed. The crude tritylcandesartan cilexetil could be recrystallized from organic solvent such as for example dichloromethane, ethyl acetate, isopropyl acetate, THF, acetone, methanol, ethanol and isopropanol or any mixture thereof.

Detritylation of tritylcandesartan cilexetil can be performed according to known procedures such as for example disclosed in WO 2007/042161, WO 2008/012371 and WO 2008/012372. Preferably the Lewis acid used in the deprotection step can be selected from the group consisting of, but not limited to, Zinc (II) trifluoromethanesulfonate, Zinc (II) acetate, tin (II)-salts such as for example SnX₂ wherein X means F, Cl, Br or I or tin (II)-salts with organic acids such as for example acetate and trifluoromethane sulfonate and BX₃ wherein X means F, Cl, Br or I. The deprotection step is performed in an organic solvent such as for example methylene chloride and methanol. In case when SnCl2 is used as Lewis acid in isolation step oxalic acid is added to enable the precipitation of tin oxalate.

During the crystallization of candesartan cilexetil alcohol selected from the group consisting of, but not limited to, methanol, ethanol, isopropanol, n-propanol and any mixtures thereof with water can be used. Preferably isopropyl alcohol is used.

The main advantage of the process for the preparation of candesartan cilexetil according to the present invention is in producing small particles of candesartan cilexetil preferably less than 25 µm, more preferably between 10 and 20µm, more preferably less than 10µm without agglomeration tendency and having the stable crystalline form I in highly economical manner.

The candesartan cilexetil prepared by the process according to the present invention can be in any known polymorph forms, particularly in polymorph form I or in amorphous form, more particularly in polymorph form I.

Candesartan cilexetil prepared by the process according to the present invention or by any process known from the prior art may be formulated into a stable solid pharmaceutical composition wherein the granules of candesartan cilexetil and at least one pharmaceutically acceptable ingredient are prepared in fluid-bed granulator and wherein candesartan cilexetil is dispersed in a solvent.

The concentration of candesartan cilexetil in the formulation, determined by %w/w calculated to the total weight of the dosage form, may be between about 1 to about 99%, particularly between about 1 to about 30% and more particularly between about 2 to about 25% determined by HPLC.

The average particle size of candesartan cilexetil may be between 1 and 50 µm, preferably between 1 and 40 µm, more preferably less than 20 µm, determined by laser diffraction method on Malvern Mastersizer 2000 instrument equiped with Hydro S dispersion unit, which is appropriate for measuring particle size distributions in the range of 20 nm to 2000µm. Isopar L is used as dispersant. Usually, 100-800mg of substance is dispersed in 5-10 ml of 2% solution of epicurone (lecithin) in isopar L to get homogenuous sample suspension. The measurement was started after addition of sample suspension into dispersion unit and homogenizing by sonication for appropriate time to brake-up agglomerates and recirculating at stirrer/pump speed rate about 50-70% of maximum speed.

As used herein, the term stable refers to candesartan cilexetil wherein the content of candesartan cilexetil is between about 85 to about 115%, particularly between about 90 to about 110% and more particularly about 95 to about 105% determined by HPLC, wherein the content uniformity of at least two samples, as for example of granulate, compression mixture, tablet or optionally, film coated tablet, is between about 75 to about 125%, particularly about 85 to about 115% and more particularly about 90 to 110% determined by HPLC, wherein the sum of all impurities is less than 0.10% determined by HPLC and wherein the sum of all impurities does not exceed 6 %w/w, more preferably 2 and the most preferably 1 %w/w determined by liquid chromatography if such a composition is stored for 7 days at 60 °C in a closed vessel.

The stable solid pharmaceutical composition according to the present invention may optionally contain one or more other active ingredients. Suitable other active ingredients can include but are not limited to other angiotensin-II-antagonists, diuretics, sympathoplegic agents, Ca-channel blockers, vasodilators, ACE inhibitor, angiotensin receptor antagonists, inhibitors of HMG-CoA reductase and any other pharmaceutically acceptable combination, particularly the other active agent is selected from the group consisting of, but not limited to, angiotensin-II-antagonist, Ca-channel blocker, diuretic, inhibitor of HMG-CoA reductase. When two or more other active ingredients are present in the stable pharmaceutical composition according to the present invention, they can be present in the same phase (in intragranular phase or in extragranular phase) as candesartan cilexetil or in the different phase as candesartan cilexetil, Additionally, candesartan cilexetil and one or more other active ingredient can be present partly in intragranular and partly in extragranular phase. The concentration of candesartan cilexetil and one or more other active ingredient may be between about 1 to about 99%, particularly between about 1 to about 30% and more particularly about 2 to about 25% determined by HPLC.

The stable solid pharmaceutical composition according to the present invention may further include one or more pharmaceutically acceptable ingredients, i.e. excipients such as for example stabilizing agents, diluents, binders, disintegrants, surfactants and lubricants. Other and further excipients can also be included. The terms » pharmaceutically acceptable ingredients« or its synonymus »pharmaceutically acceptable excipients« denotes the additives used to convert pharmacologically active compounds into pharmaceutical dosage forms suitable for administration to patients. Pharmaceutically acceptable excipients can be solid, liquid or semi-soiid and convert during the process into the state that allows prepraration of stable solid pharmaceutical composition comprising candesartan or pharmaceutically acceptable forms thereof. The pharmaceutically acceptable excipient may be present in the stable solid pharmaceutical composition according to the present invention in an amount of about 1 to about 99%, particularly from about 70 to about 99%, more particularly from about 75 to about 98%.

The particle size of excipients used in stable solid pharmaceutical composition according to the present invention are within the range of D90<750 µm, preferably D90<500, in order to ensure homogeneity of the compression mixture and homogeneous granulation and compression. D90 means that at least 90% by volume of the particles have a particle size below the specified value.

The stable solid pharmaceutical composition according to the present invention may contain one or more stabilizing agents selected from the group consisting of, but not limited to, polyols such as for example glycerol, propylene glycol and macrogols, oils/glycerides such as for example castor oil, acetylated monoglycerides, fractionated coconut oil and lanolin, organic esters such as for example triacetin, phthalate esters like polyvinyl acetate phthalate, cellulose acetate phthalate, dibutyl phthalate, diethyl phthalate, dimethyl phthalate, hypromellose phthalate and sebacetes like dibutyl sebacate and diethyl sebacate, aliphatic polyesters such as for example polylactide, polyglycolide, polylactide-co-glycolide, polycaprolactone and polylactide-co-caprolactone, polymethyl vinyl ether/maleic anhydride copolymers like Gantrez polymers, alginates such as for example sodium, potassium, ammonium, calcium and propylene glycol alginate, natural polysaccharides of marine such as for example agar and carrageenan, plant such as for example acacia, ceratonia and tragacanth or animal origin such as for example gelatin, surfactants such as for example sodium lauryl sulphate, benzalkonium chloride, cetrimide, docusate sodium, calcium stearoyl-2-lactylate, citrate esters (such as for example triethyl citrate, acetyltriethyl citrate, tributyl citrate, acetyltributyl citrate, acetyltri-2-ethylhexyl citrate), polyacrylate and polymethacrylate polymers such as for example Eudragit and Plastoid polymers, ethylene and vinyl and acetate polymers and copolymers such as for example polyethylene, polyvinyl alcohol, polyvinyl alcohol/polyvinyl acetate copolymer and ethylene vinyl acetate, various sugars such as for example sorbitol, and xylitol, various additional plasticizers such as for example glycofurol, sodium benzoate, sodium polyphosphate and sodium stearyl fumarate and other stabilizers such as menthol, amyl alcohol and similar. Preferably one or more polyol is used as a stabilizing agent, more preferably macrogols with average molecular weight between 900 and 35000, particularly between 900 and 20000, more particularly between 3000 and 9000. One or more stabilizing agent is present in an amount of about 0.1 to about 20%, particularly about 0.5 to about 15%, more particularly about 0.5 to about 10%.

The diluents used in the stable solid pharmaceutical composition according to the present invention may be selected from the group consisting of, but not limited to, microcrystalline cellulose, powdered cellulose, lactose (anhydrous and monohydrate), compressible sugar, fructose, dextrates, sugar alcohols such as mannitol, sorbitol, maltitol, xylitol, lactitol, or other sugars such as saccharose, raffinose, trehalose, fructose or mixture thereof, siliconised microcrystalline cellulose, calcium hydrogenphosphate, calcium carbonate, calcium lactate, or any mixture thereof. Preferably, at least one diluent is selected form microcrystalline cellulose, lactose and mannitol, or any mixtures thereof, more preferably from microcrystalline cellulose and lactose.

The binders used in the stable solid pharmaceutical composition according to the present invention may be selected from the group consisting of, but not limited to, polyvinylpyrrolidone, microcrystalline cellulose, hydroxyethylcellulose, hydroxypropylcellulose, hydroxypropylmethylcellulose or other cellulose ethers, starch, pregelatinised starch, or polymethacrylate, or any mixtures thereof. It is preferable to use a binder with good water solubility. In view of the practical insolubility of starch in cold water, there is a strong preference for binders with a very good solubility in cold water. There is a variety of binders that have very good solubility in water, such as for example povidone of different K-values and hydroxypropylcellulose, such as for example partially substituted poly(hydroxypropyl) ether of cellulose and/or low substituted poly(hydroxypropyl) ether of cellulose. Preferably, at least one binder is selected from polyvinylpyrrolidone and hydroxypropylcellulose.

The disintegrants used in the stable solid pharmaceutical composition according to the present invention may be selected from the group consisting of, but not limited to, crospovidone, starch, pregelatinised starch, sodium strach glycolate, microcrystalline cellulose, carboxymethylcellulose sodium (CMC-Na) or calcium (CMC-Ca), crosslinked CMC-Na, polacrilin potassium, low-substituted hydroxypropylcellulose or any mixtures thereof. Preferably, at least one disintegrant is selected from CMC-Ca, starch and low-substituted hydroxypropylcellulose.

The surfactants used in the stable solid pharmaceutical composition according to the present invention may be selected from the group consisting of, but not limited to:
*1. Anionic surfactants,* where the hydrophilic group carries a negative charge, such as for example carbonyl (RCOO⁻), sulphonate (RSO₃⁻) or sulphate (ROSO₃⁻), examples include potassium laurate, CH₃(CH₂)₁₀COO⁻K⁺, and sodium lauryl sulphate, CH₃(CH₂)ₙSO4⁻Na⁺.
*2. Cationic surfactants,* where the hydrophilic group carries a positive charge (e.g., quaternary ammonium halides, R₄N⁺Cl⁻), examples include cetrimide, a mixture consisting mainly of tetradecyl (ca. 68%), dodecyl (ca. 22%), and hexadecyltrimethylammonium bromides (ca. 7%), as well as benzalkonium chloride, a mixture of alkylbenzyldimethylammonium chlorides of the general formula [C₆H₅CH₂N⁺(CH₃)₂R]Cl⁻, where R represents a mixture of the alkyls from C₈H₁₇ to C₁₈H₃₇.
*3. Ampholytic surfactants (also called zwitterionic surfactants),* where molecule contains, or can potentially contain, both a negative and a positive charge, (e.g., the sulfobetaines, RN⁺(CH₃)₂CH₂CH₂SO₃⁻), examples include *N-*Dodecyl-*N*,*N*-Dimethylbetaine, C₁₂H₂₅N⁺(CH₃)₂CH₂COO⁻.
*4. Nonionic surfactants,* where the hydrophile carries no charge but derives its water solubility from highly polar groups such as hydroxyl or polyoxyethylene (-OCH₂CH₂O-) groups, examples include polyoxyethylated glycol monoethers (e.g. cetomacrogol), sorbitan esters (Spans^{®}) and polysorbates (Tweens^{®}), polyoxyethylene-polyoxypropylene copolymers.

The lubricants used in the stable solid pharmaceutical composition according to the present invention may be selected from the group consisting of, but not limited to, stearic acid or stearic acid salts, such as for example magnesium stearate, magnesium palmitate, magnesium oleate, hydrogenated vegetable oil, hydrogenated castor oil, talc, sodium stearyl fumarate, macrogols or mixtures thereof. Preferably, excipients include at least one lubricant selected from stearic acid, magnesium stearate, hydrogenated vegetable oil, hydrogenated castor oil and talc, more preferably from magnesium stearate, hydrogenated castor oil and talc.

Optionally, cores/tablets can be coated with conventional materials used for film coating, i.e. as described in Pharmaceutical Coating Technology (G. Cole (ed.), 1995). Film coating formulations usually contain the following components:
- polymer(s),
- plasticizer(s),
- colourant(s)/opacifier(s),
- vehicle(s).

In film coating suspension the minor quantities of flavours, surfactants and waxes can be used. The majority of the polymers used in film coating are either cellulose derivatives, such as the cellulose ethers, or acrylic polymers and copolymers. Occasionally encountered are high molecular weight polyethylene glycols, as for example macrogols, polyvinylpyrrolidone, polyvinylalcohol and waxy materials. Their function usually is to prevent bad mouth feel and/or taste and in some cases degradation, e.g. oxidation of the active ingredients and/or excipients used.

Typical cellulose ethers which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups:
- polyols (glycerol, propylene glycol, macrogols),
- organic esters (phthalate esters, dibutyl sebacetate, citrate esters, triacetin),
- oils/glycerides (castor oil, acetylated monoglycerides, fractionated coconut oil).

Colourants/opacifiers are classified into several groups:
- organic dyes and their lakes,
- inorganic colours,
- natural colours.

Vehicles are materials that performed necessary function in that that they provide the means of conveying the coating materials to the surface of the tablet or particle. The major classes of vehicles capable of being used are water, alcohols, ketones, esters, chlorinated hydrocarbons.

Different materials from each group can also be combined in defined ratios. Film coating suspensions can also be used as ready-to-make preparations which are available on the market.

Film coating dispersion can be prepared by using different solvents as for example water, alcohols, ketones, esters and chlorinated hydrocarbons, preferably water.

A composition of coating suspension (calculated on dry material) which comprises:
- 1-99%, preferably 1-95% by weight of polymer,
- 1-50%, preferably 1-40% by weight of plasticizer,
- 0.1-20%, preferably 0.1-10% by weight of colourant/opacifier,
is particularly preferred.

Ingredient described as component of the coating layer, e.g. colourants, can be incorporated also in tablet core or divided between tablet core and film coating layer.

The stable solid pharmaceutical composition according to the present invention is preferably in the form of tablets, pills, powders, lozenges, sacchets, soft and hard gelatine capsules, suppositories etc. The dosage form is preferably suitable for oral application.

The stable solid pharmaceutical composition according to the present invention is preferably formulated in a unit dosage form, each dosage containing about 1 to 100 mg, more usually about 1 to about 50 mg of candesartan cilexetil, preferably from about 2 to 32 mg. When one or more other active ingredient is present in the stable solid pharmaceutical composition according to the present invention said other active ingredient can be present in an amount of 6.25 to 50 mg, preferably in an amount of 12.5 to 25 mg. The term »unit dosage form« refers to physically discrete units suitable as unitary dosages for human objectes and other mammals, each unit containing a predetermined quantity of candesartan cilexetil calculated to produce the desired therapeutic effect, in association with a suitable pharmaceutical acceptable excipient.

In another aspect of the present invention, the present invention relates to the process for the preparation of a stable solid pharmaceutical composition comprising candesartan cilexetil wherein the granules of the active substance and at least one pharmaceutically acceptable ingredient are prepared in fluid-bed granulator and wherein candesartan cilexetil is dispersed in a solvent.

The process for the preparation of the stable solid pharmaceutical composition according to the present invention comprises the following steps:
- granulation process of the intragranular phase preparation comprising the following steps:
   o granulating a mixture of individual components which means at least one component selected from the group of one or more active ingredient and one or more pharmaceutically acceptable excipient using granulating liquid,
   o drying of the obtained mixture,
   o optionally sieving to obtain the homogeneous dry granulate,
- preparation of the extragranular phase comprising addition of at least one component selected from the group of one or more active ingredients and one or more pharmaceutically acceptable excipient, to the granulate to give compression mixture,
- compressing the compression mixture,
- optionally, applying coating.

The granulation process is performed in fluid-bed granulator. The term "fluid-bed granulator" as used herein defines different ways for performance of granulation known from the state of the art as for example in Encyclopedia of pharmaceutical technology (ed. J. Swarbrick, 3rd edition, Informa Healthcare USA Inc., 2007):
- simple fluid bed,
- tumbling fluid bed,
- spouted bed processorassisted with a draft tube.

The spraying of the granulating liquid in fluid-bed granulator could be performed form the top (top spray), bottom (bottom spray) or tangentially (tangential spray).

Candesartan cilexetil is first prepared according to a suitable synthetic process and then purified, as for example by crystallization or other means. Then, the size of the active ingredient is determined and if it is found that there are particles having average diameter more than 50 µm, then this particles of active ingredient are reduced in any of known pharmaceutical acceptable manner, such as for example milled or crunched to a smaller average particle size less than 50 µm, preferably less than 40 µm and more preferably less than 20 µm.

**Table III: Incorporation of candesartan cilexetil in the stable solid pharmaceutical composition according to the present invention**

| Embodiment | Intragranular phase | Extragranular phase |
|---|---|---|
| | Candesartan or its pharmaceutically acceptable forms | Candesartan or its pharmaceutically acceptable forms |
| A | - | + |
| B | + | - |
| C | + | + |

| | | |
|---|---|---|
| + present in intragranular and/or extragranular phase - not present in intragranular and/or extragranular phase | | |

**Table IV: Incorporation of candesartan cilexetil and other active ingredient in the stable solid pharmaceutical composition according to the present invention**

| Embodiment | Intragranular phase | | Extragranular phase | |
|---|---|---|---|---|
| | Candesartan cilexetil | Other active ingredient | Candesartan cilexetil | Other active ingredient |
| a' | - | + | + | - |
| a" | - | - | + | + |
| b' | + | + | - | - |
| b" | + | - | - | + |
| c' | + | + | + | - |
| c" | + | - | + | + |
| d' | + | + | - | + |
| d" | - | + | + | + |
| d'" | + | + | + | + |

| | | | | |
|---|---|---|---|---|
| + present in intragranular and/or extragranular phase - not present in intragranular and/or extragranular phase | | | | |

In table III, at least one component selected from the group of candesartan cilexetil and one or more pharmaceutically acceptable excipient in both intragranular and extragranular phase, is present. In embodiment A, granulate is prepared without including candesartan cilexetil cilexetil thereto. In embodiment B, a granulate is prepared which includes candesartan cilexetil. In embodiment C, candesartan cilexetil is divided between intragranular and extragranular phase. Further, pharmaceutically acceptable excipients and components of film coating can be divided between intragranular and extragranular phase and optionally, to coating layer, either individual or in the mixture of at least two components. Furthermore, at least two components can be homogenized together prior to use in technological process due to ensurance of homogeneity in the pharmaceutical formulation, e.g. homogenization of colourant(s)/opacifier(s) with at least one diluent etc.

In table IV, at least one component selected from the group of candesartan cilexetil and other active ingredient, and one or more pharmaceutically acceptable excipient in both intragranular and extragranular phase, is present. In embodiments (a'), (a") and (d"), a granulate is prepared without including candesartan cilexetil thereto. In embodiments (a"), (b") and (c"), granulate is prepared without including the other active ingredient thereto. In embodiments (b'), (b") and (d'), a granulate is prepared which includes candesartan cilexetil. In embodiments (a'), (b') and (c'), granulate is prepared which includes the other active ingredient. In embodiments (c') and (c"), candesartan cilexetil is divided between intragranular and extragranular phase. In embodments (d') and (d"), the other active ingredient is divided between intragranular and extragranular phase. In embodiment (d"'), both candesartan cilexetil and the other active ingredient are divided between intragranular and extragranular phase.

The process of granulating the mixture of individual components as defined above can be performed in a conventional fluid-bed granulation equipment by spraying of water, alcohol, water/alcohol mixture or aqueous, alcoholic or aqueous/alcoholic granulating liquid onto an excipient or mixture of excipients by conventional pharmaceutical techniques for granulation. Preferred solvent is purified water. The terms »aqueous, alcoholic or aqueous/alcoholic granulating liquid« refers to an aqueous, alcoholic or aqueous/alcoholic dispersion which contains purified water or demineralised water or lower alcohols such as for example methanol, ethanol and isopropanol and optionally at least one pharmaceutical acceptable excipient which is dispersed, suspended or dissolved in a solvent. According to the present invention candesartan or its pharmaceutically acceptable forms, preferably candesartan cilexetil is dispersed in a solvent.

Drying of the mixture obtained as described above can be performed in any of the following ways: trays, fluid-bed, microwave assist/vacuum/gas stripping (one-pot processing) or in ay other of pharmaceutical acceptable manners, preferably in fluid-bed dryer.

Sieving process can be performed by conventional known methods.

Preparation of the extragranular phase comprising addition of at least one component selected from the group of one or more active ingredient and one or more pharmaceutically acceptable excipient, to the granulate to give compression mixture, may be performed in a conventional device used for mixing of powders, such as for example motionless (passive) mixers, diffusion, biconicdiffusion, uniconic, biconic, turbular, cubic, planetary, Y-, V-shaped, low shear or high shear mixers or in any other pharmaceutically acceptable manners.

The process of compressing of the compression mixture may be performed by a conventional known methods performed on a conventional equipment such as for example on automatic rotary compressing machine.

The process of applying coating may be performed by a conventional known methods performed on a conventional equipment such as for example on automatic coating machine.

In another aspect of the present invention, there is provided a process for the preparation of stable solid pharmaceutical composition containing candesartan cilexetil, wherein the phamarceutical composition is for use in a method of treating hypertension, cardiac diseases, cerebral apoplexy or renal diseases in a patient in need thereof, and wherein the granules of the active substance and at least one pharmaceutically acceptable ingredient are prepared in fluid-bed granulator and wherein candesartan cilexetil is dispersed in a solvent.

For a more complete understanding of the instant invention, reference is made to the following illustrative examples, although it should be clearly understood that the present invention is not limited in and way thereto.

### Examples

### Example 1:

### Preparation and crystallization of 1-(cyclohexyloxycarbonyloxy)ethyl 1-((2'-cyanobiphenyl-4-yl)methyl-2-ethoxy-1H-benzo[d]imidazole-7-carboxylate

2.16 g (4 mmol) of 1-[[(cyclohexyloxy)carbonyl]oxy]ethyl 1-(4-bromobenzyl)-2-ethoxy-1*H*-benzimidazole-7-carboxylate, 1.2 g (5.74 mmol) of potassium (2-cyanophenyl)-trifluoroboronate, 20 mg (0.09 mmol) of palladium acetate, 40 mg (0.13 mmol) of tri-*o*-tolylphosphine were given into the dried flask equipped with reflux condenser. The flask was capped with a septum and filled with nitrogen in three cycles. 24 mL of ethanol and 1.2 mL of diisoproplyethylamine were added to the flask *via* a syringe. The reaction mixture was heated under reflux for 5 hours. Then, the reaction mixture was opened to the atmosphere, cooled, filtered and the solvent was evaporated under vacuum. To the residue 80 ml of isopropyl acetate and 80 ml of 1% HCl were added. The mixture was stirred and then separated. The organic phase was washed twice with 64 ml of water, dried over Na₂SO₄, and evaporated. To the concentrate, 4 ml of *tert*-butyl methyl ether were added and the mixture was stirred for 1h at room temperature and 1h at temperature below 0°C. The precipitate was collected by filtration and dried at reduced pressure for 1 h (1.6 g, 70.5% yield).
IR: 2930, 2228, 1756, 1726, 1550, 1280, 1245, 1082, 1038, 910, 764.
¹H NMR (300 MHz, DMSO, δ): 7.91 m (1H), 7.75 m (2H), 7.45-7.60 m (5H), 7.22 m (1H), 7.10 d (2H), 6.81 q (1H), 5.63 d (1H), 5.57 d (1H), 4.46-4.68 m (3H), 1.15-1.80 m (16H).

### Example 2:

### Preparation and crystallization of 1-(cyclohexyloxycarbonyloxy)ethyl 1-((2'-cyanobiphenyl-4-yl)methyl-2-ethoxy-1H-benzo[d]imidazole-7-carboxylate

### a) 2-Ethoxy-1-[(2'-cyanobiphenyl-4-yl)-methyl]-1H-benzimidazole-7-carboxylic acid

The mixture of 12.75 g (30 mmol) of ethyl 2-ethoxy-1-[(2'-cyanobiphenyl-4-yl)-methyl]-1*H-*benzimidazole-7-carboxylate, 150 ml of methanol and 37.5 ml 1M NaOH were heated and refluxed for 3-4 hours. Then the reaction mixture was cooled, the solvent was evaporated under vacuum. To the residue 150 ml of water were added. The mixture was acidified by addition of 1M HCl (42 ml). The precipitated product was collected by filtration, washed and dried at reduced pressure for 2h (11.8 g, 98%).

### b) 1-(cyclohexyloxycarbonyloxy)ethyl 1-((2'-cyanobiphenyl-4-yl)methyl-2-ethoxy-1H-benzo[d]imidazole-7-carboxylate

11.8 g (29.7 mmol) of 2-ethoxy-1-[(2'-cyanobiphenyl-4-yl)-methyl]-1*H*-benzimidazole-7-carboxylic acid, 30 ml of dimethylacetamide, 4.68g (34 mmol) of K₂CO₃ and 10.1 g (49 mmol) of (±)1-chloroethyl cyclohexyl carbonate were heated at 60°C for 3 hours. The reaction mixture was then cooled and 300 ml of isopropyl acetate and 300 ml of water were added. The phases were separated water phase was extracted again with 300 ml of isopropyl acetate. Collected organic phases were washed with 66 ml of water, dried over Na₂SO₄, filtered and concentrated to oily residue. To this residue 10 ml of methylene chloride were added and the precipitated product was filtered off and suspended in 50 ml of *tert*-butyl methyl ether. The product was collected by filtration, washed and dried at reduced pressure (10.7 g, 64%).
IR: 2930, 2227, 1755, 1726, 1550, 1280, 1245, 1082, 1038, 910, 764
¹H NMR (300 MHz, DMSO, δ): 7.91 m (1H), 7.75 m (2H), 7.45-7.60 m (5H), 7.22 m (1H), 7.10 d (2H), 6.81 q (1H), 5.63 d (1H), 5.57 d (1H), 4.46-4.68 m (3H), 1.15-1.80 m (16H).

### Example 3:

### 1-(cyclohexyloxycarbonyloxy)ethyl 1-((2'-(2H-tetrazol-5-yl)biphenvy-4-yl)methyl)-2-ethoxy-1H-benzo[d]imidazole-7-carboxylate (candesartan cilexetil)

### Step 1 - Suzuki reaction

Glassware is dried in air drier at 130°C, flask is vented with argon, and charged with 1-(cyclohexyloxycarbonyloxy)ethyl 1-(4-bromobenzyl)-2-ethoxy-1*H-*benzo[*d*]imidazole-7-carboxylate (16.4 g, 30 mmol), potassium 2-cyanophenyltrifluoroborate (7.22 g, 34.5 mmol), palladium acetate (0.00225 g), triphenylphosphine (0.078 g), diisopropylethylamine (9 ml) and ethanol (180 ml). Reaction mixture is heated at reflux temperature for 18.5 h. Mixture is cooled to room temperature and precipitate is filtered off. To filtered solution 300 ml isopropyl acetate is added and washed with 1% HCl (240 ml). Organic phase is then washed with saturated NaHCO₃ (240 ml) and water (240 ml) and dried with Na₂SO₄ (30 g). Solvent is removed under reduced pressure to give oily concentrate. To the concentrate, 45 ml of *tert*-butyl methyl ether were added and the mixture was stirred for 1 h at room temperature and 1h at temperature below 0°C. The precipitate was collected by filtration and dried at reduced pressure for 1h (13.6 g, 80.0% yield) of 1-[[(Cyclohexyloxy)carbonyl]oxy]ethyl 2-ethoxy-1-[(2'-cyanobiphenyl-4-yl)-methyl]-1*H-*benzimidazole-7-carboxylate.

### Step 2 - Cycloaddition

Flask is charged with 1-(cyclohexyloxyicarbonyloxy)ethyl 1-[(2'-cyanobiphenyl-4-yl)methyl]-2-ethoxy-1*H*-benzo[*d*]imidazol-7-carboxylate (17.8 g, 28.9 mmol) prepared according to present invention and tributyltin azide (19.2g, 57.8 mmol) in toluene (96 ml) and heated at reflux temperature. After 52.5 h reaction mixture is cooled and solvent is removed in vacuo to yield oily residue. Hexane (180 ml) is added, and stirred at room temperature for 1 h. Solvent is decanted. Product (29 g) crude candesartan cilexetil is obtained as an oil.

### Step 3 - Tritylation

Oily residue after cycloaddition (28.96 g), triethylamine (4 ml) and triphenylchloromethane (8.03 g, 28.8 mmol) are dissolved in dichloromethane (75.2 ml) and heated at reflux temperature for 1 h. After cooling the reaction mixture to room temperature silica gel (28 g) is added, stirred for a few minutes and slowly filtered off under minimal reduced pressure through filter for Pd/C, washed with dichloromethane and evaporated to oily product. *Tert*-butyl methyl ether (58 ml) is added and stirred at room temperature for 16 h. Precipitate formed was filtered off and washed with *tert*-butyl methyl ether, to yield (8.0 g, 50 % cycloaddition and tritylation together) solid tritylcandesartan cilexetil.

### Step 4: Tritylcandesartan cilexetil recrystallisation

Solid tritylcandesartan cilexetil (8.0 g) is recrystallised from mixture of dichloromethane (8 ml) and isopropyl acetate (24 ml) at room temperature. After stirring for 3 days, precipitate is filtered off under reduced pressure, washed with cooled *tert*-butyl methyl ether, and dried in vacuum drier at 40°C for 2h. White product of tritylcandesartan cilexetil (6.3 g, 87%) is obtained.

### Step 5: Detritylation

Reaction vessel is charged with tritylcandesartan cilexetil (21.0 g, 24.6 mmol), methanol (63 ml), dichloromethane (250 ml), anhydrous tin (II) chloride (3.35 g, 17.6 mmol) and heated at reflux temperature for 4h. Mixture is cooled to 30°C, water (250 ml) and 1.57 g oxalic acid are added and the precipitate is separated. After that phases in filtrate are separated and organic phase is washed with water (2x250 ml). Organic phase is dried with magnesium sulphate (18 g) and then filtered and evaporated to oily residue (22 g), which is then dissolved in isopropyl acetate (60 ml) and stirred at room temperature for 3 h. Precipitate formed is filtered off and dried in vacuum drier at 45°C for 2 h. Partly dried product candesartan cilexetil (11.7 g, 78%) is suspended for 1 h in *tert*-butyl methyl ether (107 ml), then filtered off and dried over night at room temperature.

### Step 6: Candesartan cilexetil recrystallisation

Reaction vessel is charged with candesartan cilexetil (10.4 g) and isopropanol (52 ml) and heated until complete dissolution occurs. The obtained solution is filtered, cooled to room temperature and stirred at still the same temperature for 2 h. Precipitate is filtered off, washed, and dried in air drier at 35°C for 2 h. Final product candesartan cilexetil of polymorphic form I is obtained as white crystals (9.3 g, 89.4%).

### Example 4: Synthesis of tributyltin azide

To a cooled solution of sodium azide (4.10 g, 63.0 mmol) in water (13 ml) at 8°C Bu₃SnCl (17.1 ml) is added drop wise while stirring the solution. After 2h of stirring at 8°C, dichloromethane (38 ml) is added, stirred for another 5 minutes and phases separated. Water phase is washed with dichloromethane (13 ml). Combined organic phases are washed with 10% NaCl (13 ml), and distilled water (13 ml). Organic phase is dried with sodium sulphate (6.34 g) and solvent is removed under reduced pressure to give oily product (19.2 g, 91.8%).

### Example 5: Preparation of formulations containing candesartan cilexetil

Lactose monohydrate, corn starch and optionally ferric oxide were homogeneously mixed in a chamber of fluid bed granulator (manufactured by Aeromatic) and heated up to 33°C at air flow 350 m³/h and inlet air temperature 60°C.

Hydroxypropylcellulose was dispersed in purified water in order to obtain a clear water solution of hydroxypropylcellulose. Macrogol 6000 or 8000 was added to water solution of hydroxypropylcellulose. Finally, candesartan cilexetil was added to water solution of hydroxypropylcellulose and macrogol 6000 or 8000 in order to obtain homogeneously water dispersion, i.e. granulating liquid, containing hydroxypropylcellulose, macrogol 6000 or 8000 and candesartan cilexetil by using a propeler mixer.

Granulating liquid, prepared as described above, was sprayed onto a homogeneous mixture of lactose monohydrate, starch and optionally ferric oxide at spray-rate 80-160 g/min, air-flow 350-360 m³/h, atomizing air pressure 2.5 bars and inlet air temperature 60-80°C (inlet air humidity was 1.5-2 g/kg). The temperature of the product during granulation was 26-28°C. After spraying the whole amount of granulating liquid, addition drying was performed at air-flow 350 m³/h and inlet air temperature 70°C. The temperature of the dry granulate at the end of drying was 35°C. Finally, the dry granulate was pass through a sieving machine (manufactured by Frewitt) with sieve openings 1 mm.

Loss on drying (LOD) measurement was performed on apparatus Mettler Toledo HR73 halogen moisture analyzer at 85°C for 20 minutes. To the granulate, carboxymethylcellulose sodium and magnesium stearate were added to obtain compression mixture.

The compression mixture was compressed into tablets on automatic rotary compressing machine. The theroretical weight of 4 mg, 8 mg and 16 mg compositions after tabletting was 130 mg. The theroretical weight of 32 mg composition after tabletting was 260 mg. Hardness of tablets (performed on Erweka on 20 tablets) and disintegration time of tablets (performed in purified water at 37°C) were determined.

**Table V: Composition as well as LOD (granulate, compression mixture), hardness and disintegration time of tablet samples A, B, C and D**

| | Sample A (mg) | Sample B (mg) | Sample C (mg) | Sample D (mg) |
|---|---|---|---|---|
| Candesartan cilexetil | 4.0 | 8.0 | 16.0 | 32.0 |
| Lactose monohydrate | 93.4 | 89.4 | 81.4 | 162.8 |
| Corn starch | 20.0 | 20.0 | 20.0 | 40.0 |
| Ferric oxide | - | 0.016 | 0.032 | 0.064 |
| Macrogol 6000 or | 2.6 | 2.6 | 2.6 | 5.2 |
| macrogol 8000 | | | | |
| Hydroxypropylcellulose | 4.0 | 4.0 | 4.0 | 8.0 |
| | | | | |
| Carboxymethylcellulose calcium | 5.6 | 5.6 | 5.6 | 11.2 |
| Magnesium stearate | 0.4 | 0.4 | 0.4 | 0.8 |
| | | | | |
| LOD of granulate (%) | 1.19 | 1.13 | 1.32 | 1.12 |
| LOD of compression mixture (%) | 1.63 | 1.58 | 1.75 | 1.45 |
| Hardness of tablets (N) | 48-68 | 51-65 | 49-69 | 73-105 |
| Disintegration time (min) | 6-7 | 7-8.5 | 7-7.5 | 11-12 |

**Table VI: Particle size distribution of candesartan cilexetil used in samples A, B, C and D as described above (the measurement was determined by laser diffraction method).**

| Particle size distribution | |
|---|---|
| Average particle size | 6 µm |
| 10% of particles smaller than | 1.4 µm |
| 50% of particles smalller than | 4.4 µm |
| 90%particles smaller than | 12.3 µm |

**Table VII: Content of candesartan cilexetil at initial time and content uniformity of candesartan cilexetil, performed on 10 individual tablets.**

| | | | |
|---|---|---|---|
| Content of candesartan cilexetil at initial time (%) | 99.1 | 100.3 | 99.0 |
| Content uniformity of | 101.0 | 100.1 | 99.2 |
| candesartan | 100.6 | 100.3 | 99.9 |
| (performed on 10 | 100.8 | 98.9 | 100.0 |
| individual tablets) | 100.4 | 100.3 | 98.9 |
| | 100.2 | 99.7 | 99.4 |
| | 100.0 | 99.4 | 99.8 |
| | 100.3 | 99.3 | 99.2 |
| | 100.0 | 99.0 | 99.3 |
| | 100.6 | 100.4 | 99.4 |
| | 100.6 | 100.0 | 99.6 |
| | Average: | Average: | Average: |
| | 100.5 | 99.0 | 99.5% |
| | RSD 0.3 % | RSD 0.6% | RSD 0.4 |

The process was performed on industrial scale (more than 10 kg of the granulate) and assure homogeneity of the candesartan content in the tablets (see the results of content uniformity of candesartan cilexetil, performed on 10 individual tablets of a representative sample).

The stress stability study was perfromed at storage at 60°C for 7 days. We compared results of sample D and reference product (Atacand 32 mg^{®}) at initial time (t=0) and after exposure to 60°C for 7 days. Results are presented as amount of impurity desethyl candesartan (or oxocandesartan) (Table VIII).

**Table VIII: Results of stress stability study - amount of impurity desethyl candesartan (or oxocandesartan).**

| Time of testing | Sample D | Atacand 32 mg^{®} |
|---|---|---|
| Initial time (%) | 0.03 | 0.22 |
| After storing at 60°C for 7 days (%) | 0.11 | 0.37 |

It is obvious to those skilled in art that both amount of impuritiy desethyl candesartan (or oxocandesartan) at initial time and after exposure to 60°C for 7 days of the sample D if comparing to the Atacand 32 mg^{®} show a significant advantage in both absoute amount of impuritiy desethyl candesartan (or oxocandesartan) at initial time (0.03% and 0.22% for sample D and Atacand 32 mg^{®}, subsequently) and relative increase of amount of impuritiy desethyl candesartan (or oxocandesartan) from initial time to day 7 of storage to 60°C (0.08% and 0.15% for sample D and Atacand 32 mg^{®}, subsequently).

### Example 6: Preparation of formulations containing candesartan cilexetil and hydrochlorothiazide

Lactose monohydrate, corn starch and optionally ferric oxide were homogeneously mixed in a chamber of fluid bed granulator (manufactured by Glatt) and heated up to 35°C and inlet air temperature 49°C.

Hydroxypropylcellulose was dispersed in purified water in order to obtain a clear water solution of hydroxypropylcellulose. Macrogol 6000 or 8000 was added to water solution of hydroxypropylcellulose. Finally, candesartan cilexetil was added to water solution of hydroxypropylcellulose and macrogol 6000 or 8000 in order to obtain homogeneously water dispersion, i.e. granulating liquid, containing hydroxypropylcellulose, macrogol 6000 or 8000 and candesartan cilexetil by using a propeler mixer.

Granulating liquid, prepared as described above, was sprayed onto a homogeneous mixture of lactose monohydrate, starch and optionally ferric oxide at inlet air temperature 49-55°C. The temperature of the product during granulation was 27°C. After spraying the whole amount of granulating liquid, addition drying was performed at inlet air temperature 55°C. The temperature of the dry granulate at the end of drying was 36°C. Finally, the dry granulate was pass through a sieving machine (manufactured by Frewitt) with sieve openings 1 mm.

Loss on drying (LOD) measurement was performed on apparatus Mettler Toledo HR73 halogen moisture analyzer at 85°C for 20 minutes. To the granulate, hydrochlorothiazide, carboxymethylcellulose sodium and magnesium stearate were added to obtain compression mixture.

The compression mixture was compressed into tablets on automatic rotary compressing machine. The theroretical weight of 8/12.5 mg and 16/12.5 mg compositions after tabletting was 130 mg. Hardness of tablets (performed on Erweka on 20 tablets) and disintegration time of tablets (performed in purified water at 37°C) were determined.

**Table IX: Composition as well as LOD (granulate, compression mixture), hardness and disintegration time of tablet samples E and F**

| | Sample E (mg) | Sample F (mg) |
|---|---|---|
| Candesartan cilexetil | 8.0 | 16.0 |
| Lactose monohydrate | 76.9 | 68.9 |
| Corn starch | 20.0 | 20.0 |
| Ferric oxide | - | 0.032 |
| Macrogol 6000 or macrogol 8000 | 2.6 | 2.6 |
| Hydroxypropylcellulose | 4.0 | 4.0 |
| | | |
| Hydrochlorothiazide | 12.5 | 12.5 |
| Carboxymethylcellulose calcium | 5.6 | 5.6 |
| Magnesium stearate | 0.4 | 0.4 |
| | | |
| LOD of granulate (%) | 1.46 | 1.66 |
| LOD of compression mixture (%) | 1.50 | 1.73 |
| Hardness of tablets (N) | 49-64 | 52-66 |
| Disintegration time (min) | 6-7 | 7-7.5 |

**Table X: Content of candesartan cilexetil and hydrochlorothiazide at initial time**

| | Sample E (%) | Sample F (%) |
|---|---|---|
| Content of candesartan cilexetil at initial time | 103.8 | 102.8 |
| Content of hydrochlorothiazide at initial time | 99.3 | 102.1 |

### Example 7: Alternative composition

The formulation has been prepared according to Example 6 with the same quantitative composition of the formulation, with the only difference that the amount of hydrochlorothiazide is incorporated into granulate (intragranular addition).

### Example 8: Alternative composition

The formulation has been prepared according to Example 6 with the same quantitative composition of the formulation, with the only difference that 50% w/w of the amount of hydrochlorothiazide is incorporated into granulate (intragranular addition), and the rest of hydrochlorothiazide to extragranular phase (extragranularly).

## Claims

1. Crystalline 1-(cyclohexyloxycarbonyloxy)ethyl 1-((2'-cyanobiphenyl-4-yl)methyl-2-ethoxy-1*H*-benzo[*d*]imidazole-7-carboxylate of formula (I) **characterized by** having an X-ray diffraction pattern containing the following 2-theta peaks measured using CuKα radiation: 9.1, 11.2, 13.3, 14.0, 18.4, 22.5, 23.6 ± 0.2 degrees two-theta.

2. Crystalline 1-(cyclohexyloxycarbonyloxy)ethyl 1-((2'-cyanobiphenyl-4-yl)methyl-2-ethoxy-1*H*-benzo[*d*]imidazole-7-carboxylate of formula (I) according to claim 1 **characterized by** having an X-ray diffraction pattern containing the following 2-theta peaks measured using CuKα radiation: 9.1, 11.2, 12.5, 13.3, 13.8, 14.0, 14.9, 15.4, 17.0, 18.4, 19.0, 19.8, 22.5, 23.1, 23.6, 23.8, 24.2 ± 0.2 degrees two-theta.

3. A process for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising:
a) dissolving of 1-(cyclohexyloxycarbonyloxy)ethyl 1-((2'-cyanobiphenyl-4-yl)methyl)-2-ethoxy-1*H*-benzo[*d*]imidazole-7-carboxylate in form of an oily residue in an unpolar organic solvent,
b) optionally heating the mixture to a temperature between 20 to 120°C for 0 to 24 hours, followed by cooling the solution to a temperature between -15°C and room temperature,
c) crystallization and isolating the crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate.

4. A process according to claim 3 for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising:
a) reacting 1-[[(cyclohexyloxy)carbonyl]oxy]ethyl 1-(4-bromobenzyl)-2-ethoxy-1*H*-benzimidazole-7-carboxylate with 2-cyanophenylboronic acid or its derivative in an organic solvent selected from the group consisting of alcohols, ethers or any mixtures thereof by an addition of a catalyst selected from palladium complexes to obtain 1-(cyclohexyloxycarbonyloxy)ethyl 1-((2'-cyanobiphenyl-4-yl)methyl)-2-ethoxy-1*H*-benzo[*d*]imidazole-7-carboxylate in the form of a solution or an oily residue,
b) dissolving of the obtained 1-(cyclohexyloxycarbonyloxy)ethyl 1-((2'-cyanobiphenyl-4-yl)methyl)-2-ethoxy-1*H*-benzo[*d*]imidazole-7-carboxylate in an unpolar organic solvent,
c) optionally heating the mixture to a temperature between 20 to 120°C for 0 to 24 hours, followed by cooling the solution to a temperature between -15°C and room temperature,
d) crystallization and isolating the crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate.

5. A process for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate according to claims 3 and 4 wherein the unpolar organic solvent is selected from the group consisting of ethers such as for example diethyl ether, diisopropyl ether, *tert*-butyl methyl ether and like, cyclohexane, alkanes, halogenated alkanes, or any mixtures thereof.

6. A process according to claim 4 for the preparation of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate comprising:
a) reacting an ester of 2-Ethoxy-1-[(2'-cyanobiphenyl-4-yl)-methyl]-1H-benzimidazole-7-carboxylic acid with a base selected from the group consisting of NaOH, KOH, LiOH, Mg(OH)₂ or Ca(OH)₂ to form a corresponding salt of 2-Ethoxy-1-[(2'-cyanobiphenyl-4-yl)-methyl]-1H-benzimidazole-7-carboxylic acid,
b) reacting a salt of 2-Ethoxy-1-[(2'-cyanobiphenyl-4-yl)-methyl]-1H-benzimidazole-7-carboxylic acid with an acid selected from the group consisting of HCl, H₂SO₄, H₃PO₄ to form 2-Ethoxy-1-[(2'-cyanobiphenyl-4-yl)-methyl]-1H-benzimidazole-7-carboxylic acid,
c) reacting 2-Ethoxy-1-[(2'-cyanobiphenyl-4-yl)-methyl]-1H-benzimidazole-7-carboxylic acid with 1-haloethyl cyclohexyl carbonate selected from the group consisting of chloroethyl, iodoethyl, bromoethyl cyclohexyl carbonate to form 1-(cyclohexyloxycarbonyloxy)ethyl 1-((2'-cyanobiphenyl-4-yl)methyl)-2-ethoxy-1H-benzo[d]imidazole-7- carboxylate,
d) dissolving of the obtained 1-(cyclohexyloxycarbonyloxy)ethyl 1-((2'-cyanobiphenyl-4-yl)methyl)-2-ethoxy-1*H-*benzo[*d*]imidazole-7-carboxylate in an unpolar organic solvent,
e) optionally heating the mixture to a temperature between 20 to 120°C for 0 to 24 hours, followed by cooling the solution to a temperature between -15°C and room temperature,
f) crystallization and isolating the .crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate.

7. Use of crystalline form of 1-(cyclohexyloxycarbonyloxy)ethyl 1-((2'-cyanobiphenyl-4-yl)methyl)-2-ethoxy-1*H*-benzo[*d*]imidazole-7-carboxylate for the preparation of candesartan cilexetil or a solid pharmaceutical composition containing candesartan cilexetil.

8. A process for the preparation of candesartan cilexetil comprising:
a) preparation of a crystalline form of 1-(cyclohexyloxycarbonyloxy)ethyl 1-((2'-cyanobiphenyl-4-yl)methyl-2-ethoxy-1*H*-benzo[*d*]imidazole-7-carboxylate according to a process as claimed in any of claims 3-6,
b) cycloaddition of an azide on said crystalline 1-(cyclohexyloxycarbonyloxy)ethyl 1-((2'-cyanobiphenyl-4-yl)methyl-2-ethoxy-1*H*-benzo[*d*]imidazole-7-carboxylate to obtain crude candesartan cilexetil,
c) purification of crude candesartan cilexetil,
d) crystallization of candesartan cilexetil.

9. A process for the preparation of candesartan cilexetil according to claim 8 wherein the cycloaddition is performed in an organic solvent selected from the group consisting of toluene and xylene at elevated temperature between 100 and 140°C.

10. A process for the preparation of candesartan cilexetil according to claim 8 wherein the purification of crude candesartan cilexetil is performed by purification on column chromatography.

11. A process for the preparation of candesartan cilexetil according to claim 8 wherein the purification of crude candesartan cilexetil is performed by transforming candesartan cilexetil to tritylcandesartan cilexetil and subsequent detritylation.

12. A process for the preparation of candesartan cilexetil according to claim 8 wherein the crystallization of candesartan cilexetil is carried out in an alcohol selected from the group consisting of methanol, ethanol, isopropanol, n-propanol and any mixtures thereof with water.

13. Process for the preparation of a stable solid pharmaceutical composition containing candesartan cilexetil, comprising the preparation of candesartan cilexetil according to any of claims 8 to 12 and formulating the thus obtained candesartan cilexetil into a solid pharmaceutical composition.

14. The process according to claim 13, comprising the step of preparing granules of candesartan cilexetil and at least one pharmaceutically acceptable ingredient in a fluid-bed granulator, wherein candesartan cilexetil is dispersed in a solvent.

15. The process according to claim 14, wherein the pharmaceutical composition is for use in a method of treating hypertension, cardiac diseases, cerebral apoplexy or renal diseases in a patient in need thereof.

## Patentansprüche

1. Kristallines 1-(Cyclohexyloxycarbonyloxy)ethyl-1-((2'-cyanobiphenyl-4-yl)methyl)-2-ethoxy-1H-benzo[d]imidazol-7-carboxylat der Formel (I), **dadurch gekennzeichnet, dass** es ein Röntgendiffraktionsmuster hat, das die folgenden 2-Theta-Peaks enthält, die mittels CuKα-Strahlung gemessen wurden: 9,1, 11,2, 13,3, 14,0, 18,4, 22,5, 23,6 ± 0,2° 2-Theta.

2. Kristallines 1-(Cyclohexyloxycarbonyloxy)ethyl-1-((2'-cyanobiphenyl-4-yl)methyl)-2-ethoxy-1H-benzo[d]imidazol-7-carboxylat der Formel (I) gemäß Anspruch 1, **dadurch gekennzeichnet, dass** es ein Röntgendiffraktionsmuster hat, das die folgenden 2-Theta-Peaks enthält, die mittels CuKα-Strahlung gemessen wurden: 9,1, 11,2, 12,5, 13,3, 13,8, 14,0, 14,9, 15,4, 17,0, 18,4, 19,0, 19,8, 22,5, 23,1, 23,6, 23,8, 24,2 ± 0,2° 2-Theta.

3. Verfahren zur Herstellung einer kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat, umfassend:
a) das Auflösen von 1-(Cyclohexyloxycarbonyloxy)ethyl-1-((2'-cyanobiphenyl-4-yl)methyl)-2-ethoxy-1H-benzo[d]imidazol-7-carboxylat in Form eines öligen Rückstands in einem unpolaren organischen Lösungsmittel,
b) das wahlweise Erhitzen der Mischung auf eine Temperatur zwischen 20 und 120°C für 0 bis 24 Stunden, gefolgt vom Abkühlen der Lösung auf eine Temperatur zwischen -15°C und Raumtemperatur,
c) das Kristallisieren und Isolieren der kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat.

4. Verfahren gemäß Anspruch 3 zur Herstellung einer kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat, umfassend:
a) das Umsetzen von 1-[[(Cyclohexyloxy)carbonyl]oxy]ethyl-1-(4-brombenzyl)-2-ethoxy-1H-benzimidazol-7-carboxylat mit 2-Cyanophenylboronsäure oder dessen Derivat in einem organischen Lösungsmittel, ausgewählt aus der Gruppe bestehend aus Alkoholen, Ethern und beliebigen Mischungen davon, durch Zugabe eines Katalysators, ausgewählt aus Palladiumkomplexen, um 1-(Cyclohexyloxycarbonyloxy)ethyl-1-((2'-cyanobiphenyl-4-yl)methyl)-2-ethoxy-1H-benzo[d]imidazol-7-carboxylat in Form einer Lösung oder eines öligen Rückstands zu erhalten,
b) das Auflösen des erhaltenen 1-(Cyclohexyloxycarbonyloxy)ethyl-1-((2'-cyanobiphenyl-4-yl)methyl)-2-ethoxy-1H-benzo[d]imidazol-7-carboxylats in einem unpolarorganischen Lösungsmittel,
c) das wahlweise Erhitzen der Mischung auf eine Temperatur zwischen 20 und 120°C für 0 bis 24 Stunden, gefolgt vom Abkühlen der Lösung auf eine Temperatur zwischen -15°C und Raumtemperatur,
d) das Kristallisieren und Isolieren der kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat.

5. Verfahren zur Herstellung einer kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat gemäß den Ansprüchen 3 und 4, wobei das unpolare organische Lösungsmittel ausgewählt aus der Gruppe bestehend aus Ethern, wie z.B. Diethylether, Diisopropylether, tert-Butylmethylether und dgl., Cyclohexan, Alkanen, halogenierten Alkanen oder beliebigen Mischungen davon.

6. Verfahren gemäß Anspruch 4 zur Herstellung einer kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat, umfassend:
a) das Umsetzen eines Esters der 2-Ethoxy-1-[(2'-cyanobiphenyl-4-yl)methyl]-1H-benzimidazol-7-carbonsäure mit einer Base, ausgewählt aus der Gruppe bestehend aus NaOH, KOH, LiOH, Mg(OH)₂ oder Ca(OH)₂, um ein entsprechendes Salz der 2-Ethoxy-1-[(2'-cyanobiphenyl-4-yl)methyl]-1H-benzimidazol-7-carbonsäure zu bilden,
b) das Umsetzen eines Salzes der 2-Ethoxy-1-[(2'-cyanobiphenyl-4-yl)methyl]-1H-benzimidazol-7-carbonsäure mit einer Säure, ausgewählt aus der Gruppe bestehend aus HCl, H₂SO₄, H₃PO₄, um 2-Ethoxy-1-[(2'-cyanobiphenyl-4-yl)methyl]-1H-benzimidazol-7-carbonsäure zu bilden,
c) das Umsetzen der 2-Ethoxy-1-[(2'-cyanobiphenyl-4-yl)methyl]-1H-benzimidazol-7-carbonsäure mit einem 1-Haloethylcyclohexylcarbonat, ausgewählt aus der Gruppe bestehend aus Chlorethyl-, Jodethyl- und Bromethylcyclohexylcarbonat, um 1-(Cyclohexyloxycarbonyloxy)ethyl-1-((2'-cyanobiphenyl-4-yl)methyl)-2-ethoxy-1H-benzo[d]imidazol-7-carboxylat zu bilden,
d) das Auflösen des erhaltenen 1-(Cyclohexyloxycarbonyloxy)ethyl-1-((2'-cyanobiphenyl-4-yl)methyl)-2-ethoxy-1H-benzo[d]imidazol-7-carboxylats in einem unpolaren organischen Lösungsmittel,
e) das wahlweise Erhitzen der Mischung auf eine Temperatur zwischen 20 und 120°C für 0 bis 24 Stunden, gefolgt vom Abkühlen der Lösung auf eine Temperatur zwischen -15°C und Raumtemperatur,
f) das Kristallisieren und Isolieren der kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat.

7. Verwendung einer kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat zur Herstellung von Candesartancilexetil oder einer festen pharmazeutischen Zusammensetzung, die Candesartancilexetil enthält.

8. Verfahren zur Herstellung von Candesartancilexetil, umfassend:
a) das Herstellen einer kristallinen Form von 1-(Cyclohexyloxycarbonyloxy)ethyl-1-((2'-cyanobiphenyl-4-yl)methyl)-2-ethoxy-1H-benzo[d]imidazol-7-carboxylat gemäß einem Verfahren wie in einem der Ansprüche 3 bis 6 beansprucht,
b) die Cycloaddition eines Azids an das kristalline 1-(Cyclohexyloxycarbonyloxy)ethyl-1-((2'-cyanobiphenyl-4-yl)methyl)-2-ethoxy-1H-benzo[d]imidazol-7-carboxylat, um Roh-Candesartancilexetil zu erhalten,
c) das Aufreinigen des Roh-Candesartancilexetils,
d) das Kristallisieren von Candesartancilexetil.

9. Verfahren zur Herstellung von Candesartancilexetil gemäß Anspruch 8, wobei die Cycloaddition in einem organischen Lösungsmittel, das ausgewählt ist aus der Gruppe bestehend aus Toluol und Xylol, bei einer erhöhten Temperatur zwischen 100 und 140°C durchgeführt wird.

10. Verfahren zur Herstellung von Candesartancilexetil gemäß Anspruch 8, wobei die Reinigung des Roh-Candesartancilexetils durch Aufreinigung mittels Säulenchromatografie durchgeführt wird.

11. Verfahren zur Herstellung von Candesartancilexetil gemäß Anspruch 8, wobei die Reinigung des Roh-Candesartancilexetils mittels Überführung des Candesartancilexetils in Tritylcandesartancilexetil und nachfolgende Detritylierung geführt wird.

12. Verfahren zur Herstellung von Candesartancilexetil gemäß Anspruch 8, wobei das Kristallisieren von Candesartancilexetil in einem Alkohol durchgeführt wird, der ausgewählt ist aus der Gruppe bestehend aus Methanol, Ethanol, Isopropanol, n-Propanol und beliebigen Mischungen davon mit Wasser.

13. Verfahren zur Herstellung einer stabilen festen pharmazeutischen Zusammensetzung, enthaltend Candesartancilexetil, umfassend die Herstellung von Candesartancilexetil gemäß einem der Ansprüche 8 bis 12 und die Formulierung des so erhaltenen Candesartancilexetils in eine feste pharmazeutische Zusammensetzung.

14. Verfahren gemäß Anspruch 13, umfassend den Schritt der Herstellung eines Granulats aus Candesartancilexetil und mindestens einem pharmazeutisch akzeptablen Bestandteil in einem Fließbettgranulator, wobei das Candesartancilexetil in einem Lösungsmittel dispergiert wird.

15. Verfahren gemäß Anspruch 14, wobei die pharmazeutische Zusammensetzung zur Verwendung in einem Verfahren zur Behandlung von Bluthochdruck, Herzerkrankungen, Hirnschlag oder Nierenerkrankungen in einem Patienten, der dieser bedarf, ist.

## Revendications

1. 1-(Cyclohexyloxycarbonyloxy)éthyl 1-((2'-cyanobiphényl-4-yl)méthyl)-2-éthoxy-1*H*-benzo[*d*]imidazole-7-carboxylate cristallin de formule (I), **caractérisé en ce qu'**il a un diagramme de diffraction des rayons X contenant les pics 2-théta suivants mesurés en utilisant un rayonnement CuKα : 9,1, 11,2, 13,3, 14,0, 18,4, 22,5, 23,6 ± 0,2 degrés deux-théta.

2. 1-(Cyclohexyloxycarbonyloxy)éthyl 1-((2'-cyanobiphényl-4-yl)méthyl)-2-éthoxy-1*H*-benzo[*d*]imidazole-7-carboxylate cristallin de formule (I) selon la revendication 1, **caractérisé en ce qu'**il a un diagramme de diffraction des rayons X contenant les pics 2-théta suivants mesurés en utilisant un rayonnement CuKα : 9,1, 11,2, 12,5, 13,3, 13,8, 14,0, 14,9, 15,4, 17,0, 18,4, 19,0, 19,8, 22,5, 23,1, 23,6, 23,8, 24,2 ± 0,2 degrés deux-théta.

3. Procédé pour la préparation d'une forme cristalline de 1-(cyclohexyloxycarbonyloxy)éthyl 1-((2'-cyanobiphényl-4-yl)méthyl)-2-éthoxy-1*H*-benzo[*d*]imidazole-7-carboxylate comprenant :
a) la dissolution de 1-(cyclohexyloxycarbonyloxy)éthyl 1-((2'-cyanobiphényl-4-yl)méthyl)-2-éthoxy-1*H*-benzo[*d*]imidazole-7-carboxylate sous la forme d'un résidu huileux dans un solvant organique non polaire,
b) optionnellement le chauffage du mélange jusqu'à une température comprise entre 20 et 120 °C pendant 0 à 24 heures, suivi du refroidissement de la solution jusqu'à une température entre -15 °C et la température ambiante,
c) la cristallisation et l'isolement de la forme cristalline du 1-(cyclohexyloxycarbonyloxy)éthyl 1-((2'-cyanobiphényl-4-yl)méthyl)-2-éthoxy-1*H*-benzo[*d*]imidazole-7-carboxylate.

4. Procédé selon la revendication 3 pour la préparation d'une forme cristalline de 1-(cyclohexyloxycarbonyloxy)éthyl 1-((2'-cyanobiphényl-4-yl)méthyl)-2-éthoxy-1*H*-benzo[*d*]imidazole-7-carboxylate comprenant :
a) la mise en réaction de 1-[[(cyclohexyloxy)carbonyl]oxy]éthyl 1-(4-bromobenzyl)-2-éthoxy-1*H*-benzimidazole-7-carboxylate avec de l'acide 2-cyanophénylboronique ou son dérivé dans un solvant organique sélectionné dans le groupe constitué par des alcools, des éthers ou de quelconques mélanges de ceux-ci par une addition de catalyseur sélectionné parmi des complexes de palladium pour obtenir du 1-(cyclohexyloxycarbonyloxy)éthyl 1-((2'-cyanobiphényl-4-yl)méthyl)-2-éthoxy-1*H*-benzo[*d*]imidazole-7-carboxylate sous la forme d'une solution ou d'un résidu huileux,
b) la dissolution du 1-(cyclohexyloxycarbonyloxy)éthyl 1-((2'-cyanobiphényl-4-yl)méthyl)-2-éthoxy-1*H*-benzo[*d*]imidazole-7-carboxylate obtenu dans un solvant organique non polaire,
c) optionnellement le chauffage du mélange jusqu'à une température comprise entre 20 et 120 °C pendant 0 à 24 heures, suivi d'un refroidissement de la solution jusqu'à une température entre -15 °C et la température ambiante,
d) la cristallisation et l'isolement de la forme cristalline du 1-(cyclohexyloxycarbonyloxy)éthyl 1-((2'-cyanobiphényl-4-yl)-méthyl)-2-éthoxy-1*H*-benzo[*d*]imidazole-7-carboxylate.

5. Procédé pour la préparation d'une forme cristalline de 1-(cyclohexyloxycarbonyloxy)éthyl 1-((2'-cyanobiphényl-4-yl)méthyl)-2-éthoxy-1*H*-benzo[*d*]imidazole-7-carboxylate selon les revendications 3 et 4, dans lequel le solvant organique non polaire est sélectionné dans le groupe constitué par des éthers tels que, par exemple, le diéthyléther, le diisopropyléther, le *tert*-butylméthyléther et autres du même type, le cyclohexane, des alcanes, des alcanes halogénés, ou de quelconques mélanges de ceux-ci.

6. Procédé selon la revendication 4 pour la préparation d'une forme cristalline de 1-(cyclohexyloxycarbonyloxy)éthyl 1-((2'-cyanobiphényl-4-yl)méthyl)-2-éthoxy-1*H*-benzo[*d*]imidazole-7-carboxylate comprenant :
a) la mise en réaction d'un ester de l'acide 2-éthoxy-1-[(2'-cyanobiphényl-4-yl)-méthyl]-1H-benzimidazole-7-carboxylique avec une base sélectionnée dans le groupe constitué par NaOH, KOH, LiOH, Mg(OH)₂ ou Ca(OH)₂ pour former un sel correspondant de l'acide 2-éthoxy-1-[(2'-cyanobiphényl-4-yl)-méthyl]-1H-benzimidazole-7-carboxylique,
b) la mise en réaction d'un sel de l'acide 2-éthoxy-1-[(2'-cyanobiphényl-4-yl)-méthyl]-1H-benzimidazole-7-carboxylique avec un acide sélectionné dans le groupe constitué par HCl, H₂SO₄, H₃PO₄ pour former de l'acide 2-éthoxy-1-[(2'-cyanobiphényl-4-yl)-méthyl]-1H-benzimidazole-7-carboxylique,
c) la mise en réaction de l'acide 2-éthoxy-1-[(2'-cyanobiphényl-4-yl)-méthyl]-1H-benzimidazole-7-carboxylique avec du 1-halogénoéthyl cyclohexyl carbonate sélectionné dans le groupe constitué par du chloroéthyl, de l'iodoéthyl, du bromoéthyl cyclohexyl carbonate pour former du 1-(cyclohexyloxycarbonyloxy)éthyl 1-((2'-cyanobiphényl-4-yl)-méthyl)-2-éthoxy-1*H*-benzo[*d*]imidazole-7-carboxylate,
d) la dissolution du 1-(cyclohexyloxycarbonyloxy)éthyl 1-((2'-cyanobiphényl-4-yl)méthyl)-2-éthoxy-1*H*-benzo[*d*]imidazole-7-carboxylate obtenu dans un solvant organique non polaire,
e) optionnellement le chauffage du mélange jusqu'à une température comprise entre 20 et 120 °C pendant 0 à 24 heures, suivi d'un refroidissement de la solution jusqu'à une température entre -15 °C et la température ambiante,
f) la cristallisation et l'isolement de la forme cristalline du 1-(cyclohexyloxycarbonyloxy)éthyl 1-((2'-cyanobiphényl-4-yl)-méthyl)-2-éthoxy-1*H*-benzo[*d*]imidazole-7-carboxylate.

7. Utilisation d'une forme cristalline du 1-(cyclohexyloxycarbonyloxy)éthyl 1-((2'-cyanobiphényl-4-yl)méthyl)-2-éthoxy-1*H*-benzo[*d*]imidazole-7-carboxylate pour la préparation de candesartan cilexetil ou d'une composition pharmaceutique solide contenant du candesartan cilexetil.

8. Procédé pour la préparation de candesartan cilexetil comprenant :
a) la préparation d'une forme cristalline de 1-(cyclohexyloxycarbonyloxy)éthyl 1-((2'-cyanobiphényl-4-yl)méthyl)-2-éthoxy-1*H*-benzo[*d*]imidazole-7-carboxylate selon un procédé tel que revendiqué dans l'une quelconque des revendications 3 à 6,
b) la cycloaddition d'un azide sur ledit 1-(cyclohexyloxycarbonyloxy)éthyl 1-((2'-cyanobiphényl-4-yl)méthyl)-2-éthoxy-1*H*-benzo[*d*]imidazole-7-carboxylate cristallin pour obtenir du candesartan cilexetil brut,
c) la purification de candesartan cilexetil brut,
d) la cristallisation de candesartan cilexetil.

9. Procédé pour la préparation de candesartan cilexetil selon la revendication 8, dans lequel la cycloaddition est effectuée dans un solvant organique sélectionné dans le groupe constitué par le toluène et le xylène à une température élevée entre 100 et 140 °C.

10. Procédé pour la préparation de candesartan cilexetil selon la revendication 8, dans lequel la purification du candesartan cilexetil brut est effectuée au moyen d'une purification par chromatographie sur colonne.

11. Procédé pour la préparation de candesartan cilexetil selon la revendication 8, dans lequel la purification du candesartan cilexetil brut est effectuée par transformation de candesartan cilexetil en tritylcandesartan cilexetil et détritylation ultérieure.

12. Procédé pour la préparation de candesartan cilexetil selon la revendication 8, dans lequel la cristallisation du candesartan cilexetil est réalisée dans un alcool sélectionné dans le groupe constitué par le méthanol, l'éthanol, l'isopropanol, le *n*-propanol et de quelconques mélanges de ceux-ci avec de l'eau.

13. Procédé pour la préparation d'une composition pharmaceutique solide stable contenant du candesartan cilexetil, comprenant la préparation de candesartan cilexetil selon l'une quelconque des revendications 8 à 12 et la formulation du candesartan cilexetil ainsi obtenu dans une composition pharmaceutique solide.

14. Procédé selon la revendication 13, comprenant l'étape consistant à préparer des granulés de candesartan cilexetil et d'au moins un ingrédient pharmaceutiquement acceptable dans un granulateur en lit fluidisé, dans lequel le candesartan cilexetil est dispersé dans un solvant.

15. Procédé selon la revendication 14, dans lequel la composition pharmaceutique est destinée à être utilisée dans une méthode de traitement de l'hypertension, de maladies cardiaques, de l'apoplexie cérébrale ou de maladies rénales chez un patient qui en a besoin.
